# EUROPEAN PATENT APPLICATION

(11) **EP 4 755 212 A1**
(43) Date of publication of application: **10.06.2026**
(21) Application number: 23954043.8
(22) Date of filing: 14.12.2023
(51) Int. Cl.: A24F 40/10, A24F 40/44, A24F 40/48, A61M 15/06

(54) **ATOMIZING CORE ASSEMBLY AND ASSEMBLING METHOD THEREFOR, ATOMIZER, AND ATOMIZATION DEVICE**

(30) Priority: 25.09.2023 CN 202311243091
(71) Applicant: Imiracle (HK) Limited, Kowloon, Hong Kong 999077 (HK)
(72) Inventor: ZUO, Qing, Hong Kong 999077 (CN); FU, Yao, Hong Kong 999077 (CN); DUAN, Liwu, Hong Kong 999077 (CN)
(74) Representative: Vitina, Maruta
(86) International application number: PCT/CN2023/138828
(87) International publication number: WO 2025/065911

(57) **Abstract**

The present disclosure relates to an atomization core assembly and an assembly method therefor, an atomizer, and an atomization device. The atomization core assembly includes: a porous support, an atomization core, and a sealing sleeve. The porous support is provided with a mounting cavity, an outer sidewall, and micropores configured to allow atomization liquid to permeate from the outer sidewall into the mounting cavity. The atomization core is disposed in the mounting cavity, and a hollow atomization channel is formed inside. The sealing sleeve is sleeved on an outer periphery of the porous support and encloses the atomization core therein, and a side wall of the sealing sleeve is provided with a through liquid inlet hole. The atomization core assembly exhibits improved leak-resistant performance.

## Description

The present disclosure claims priority to the prior disclosure filed on September 25, 2023, entitled "ATOMIZATION CORE ASSEMBLY AND ASSEMBLY METHOD THEREFOR, ATOMIZER, AND ATOMIZATION DEVICE", with application number 202311243091.7, the content of which is incorporated herein by reference.

### TECHNICAL FIELD

The present disclosure relates to the technical field of electronic atomization technology, and specifically to an atomization core assembly and an assembly method therefor, an atomizer, and an atomization device.

### BACKGROUND

Aerosol-generating devices and electronic devices for atomizing non-nicotine media (such as healthcare drugs and therapeutic drugs) can be collectively referred to as atomizers. An atomizer is a product that can heat and atomize atomization liquid into gas. In an existing technology, an atomizer generally includes a housing and an atomization core. The atomization core includes a ceramic atomization core and a cotton atomization core. However, the ceramic atomization core generally provides a poorer flavor experience compared to the cotton atomization core. In the case that the cotton atomization core is used, a stainless-steel tube is often used as a support. The atomization liquid can only pass through the support via e-liquid guide holes in the support to enter the cotton atomization core. An insufficient number of e-liquid guide holes will lead to inadequate e-liquid supply, affecting the flavor profile of the atomizer. Conversely, too many e-liquid guide holes may reduce anti-leakage performance of the atomizer. Therefore, a pressing issue that needs to be addressed is how to improve the anti-leakage performance while ensuring the flavor profile of the atomizer.

### SUMMARY

In view of the above, the present disclosure provides an atomization core assembly and an assembly method therefor, an atomizer, and an atomization device, where the atomization core assembly exhibits improved leak-resistant performance.

The present disclosure provides an atomization core assembly, which includes: a porous support, an atomization core, and a sealing sleeve. The porous support is provided with a mounting cavity, an outer sidewall, and micropores configured to allow atomization liquid to permeate from the outer sidewall into the mounting cavity. The atomization core is disposed in the mounting cavity, and a hollow atomization channel is formed inside. The sealing sleeve is sleeved on an outer periphery of the porous support and encloses the atomization core therein, and a side wall of the sealing sleeve is provided with a through liquid inlet hole.

Furthermore, an end of the porous support at which the sealing sleeve is mounted is further provided with a notch, the notch penetrating through the outer sidewall and communicating with the mounting cavity; and the atomization core includes a liquid guide member and a heating member, and at least a portion of the liquid guide member extends into the notch. Furthermore, the liquid guide member is configured as a sheet-shaped structure. The sheet-shaped liquid guide member is enclosed to form the atomization channel, with at least one end located within the notch; an outer circumferential surface of the heating member is attached to an inner circumferential surface of the liquid guide member; and/or the porous support is configured as a porous ceramic support.

Furthermore, the mounting cavity of the porous support is provided with an air inlet hole on a side facing away from the notch, the sealing sleeve is provided with an air outlet hole on a side proximate to the notch, and the air inlet hole communicates with the air outlet hole. Furthermore, orthogonal projections of the liquid inlet hole and the notch on the outer sidewall do not overlap.

Furthermore, the sealing sleeve includes a cylindrical portion, a connecting portion, and a surrounding portion which are sequentially connected. The cylindrical portion and the surrounding portion are spaced apart and disposed on the same side of the connecting portion. The surrounding portion is disposed to surround a part of an outer periphery of the cylindrical portion. The cylindrical portion is sleeved on the outer periphery of the porous support, and the cylindrical portion, the connecting portion, and the surrounding portion together enclose to form an insertion slot.

The present disclosure further provides an assembly method for an atomization core assembly, which includes: providing an atomization core and a rod body, and wrapping the atomization core around an outer periphery of the rod body; providing a porous support having a mounting cavity, and inserting the rod body wrapped with the atomization core into the mounting cavity; and providing a sealing sleeve, sleeving the sealing sleeve onto an outer periphery of the porous support, and pulling out the rod body.

Furthermore, the atomization core includes a liquid guide member, which is configured as a sheet-shaped structure, and the porous support is provided with a notch; the wrapping the atomization core around an outer periphery of the rod body includes: winding the sheet-shaped liquid guide member around the outer periphery of the rod body such that two ends of the sheet-shaped liquid guide member are attached to each other; and the inserting the rod body wrapped with the atomization core into the mounting cavity includes: aligning the two ends of the sheet-shaped liquid guide member with the notch and inserting the sheet-shaped liquid guide member into the mounting cavity; and cutting off a portion of an end portion of the sheet-shaped liquid guide member that extends out of the notch.

Furthermore, the porous support is configured as a porous ceramic support.

The present disclosure further provides an atomizer, which includes the atomization core assembly provided by the present disclosure.

Furthermore, the atomizer further includes a base and an air guide tube. The air guide tube is sleeved on a part of the outer periphery of the sealing sleeve, the base is provided with a stepped recess, the sealing sleeve of the atomization core assembly is at least partially located within the stepped recess, and the sealing sleeve is configured to achieve a sealed connection between the base and the air guide tube.

Furthermore, the atomizer further includes a housing, the housing includes a bottom housing, the housing is mounted around the outer periphery of the base and the air guide tube, the bottom housing is disposed on a side of the base and the air guide tube, the bottom housing is provided with an air vent hole, the base further includes a blocking portion, the blocking portion is disposed on a surface of the stepped recess facing the atomization core assembly, and an orthogonal projection of the air vent hole onto the surface of the stepped recess facing the atomization core assembly falls within a range of the blocking portion, and the blocking portion is configured to prevent liquid from dripping into the air vent hole.

The present disclosure further provides an atomization device, which includes the atomizer provided by the present disclosure.

In the present disclosure, the side wall of the sealing sleeve is provided with a through liquid inlet hole. The porous support is provided with micropores configured to allow atomization liquid to permeate from the outer sidewall into the mounting cavity. When the atomization core assembly is assembled into the atomizer, the atomization liquid enters the porous support from the liquid inlet hole of the sealing sleeve and infiltrates the atomization core through the micropores. The atomization core heats and atomizes the atomization liquid into vapor, which flows out of the atomization core assembly through the atomization channel to deliver the atomized vapor to a target object. Furthermore, the present disclosure provides a porous support that is sleeved on the outer periphery of the atomization core. The porous support is provided with a plurality of micropores, which allow the atomization liquid to permeate from the outer sidewall to the mounting cavity, thereby infiltrating the atomization core for atomization. Compared to the solution of using a stainless-steel support sleeved on the outer periphery of the atomization core, the porous support provided by the present disclosure does not require e-liquid guide holes, which is beneficial to improving the anti-leakage performance of the porous support and, in turn, the anti-leakage performance of the atomization core assembly. In addition, the sealing sleeve is sleeved around the outer periphery of the porous support, preventing the atomization liquid from directly infiltrating the atomization core through a gap between the porous support and the sealing sleeve. This improves the anti-leakage performance of the atomizer and also facilitates more uniform penetration of the atomization liquid into the atomization core, which enhances the flavor profile of the vapor atomized by the atomization core and improves user experience for the target object. The atomizer provided by the present disclosure utilizes a sealing sleeve to enhance the anti-leakage performance of the atomizer. The processing and assembly costs of the sealing sleeve are low, which helps to reduce the processing and assembly costs of the atomizer.

### BRIEF DESCRIPTION OF THE DRAWINGS

To more clearly illustrate the technical solutions of the embodiments of the present disclosure, the following briefly describes the drawings required for use in the embodiments. Clearly, the drawings described below represent some embodiments of the present disclosure. Persons skilled in the art can derive other drawings based on these drawings without creative efforts.
FIG. 1 is a schematic structural diagram of an atomization core assembly according to an embodiment of the present disclosure;
FIG. 2 is an exploded structural diagram of an atomization core assembly according to an embodiment of the present disclosure;
FIG. 3 is a schematic structural diagram of a porous support according to an embodiment of the present disclosure;
FIG. 4 is an enlarged view of a porous support according to an embodiment of the present disclosure taken within the dashed box A in FIG. 3;
FIG. 5 is a schematic structural diagram of an atomizer according to an embodiment of the present disclosure;
FIG. 6 is a schematic top view of an atomizer according to an embodiment of the present disclosure;
FIG. 7 is a cross-sectional view of an atomizer according to an embodiment of the present disclosure taken along the line B-B in FIG. 6;
FIG. 8 is a schematic structural diagram of a portion of an atomizer according to an embodiment of the present disclosure;
FIG. 9 is a schematic structural diagram of a sealing sleeve according to an embodiment of the present disclosure;
FIG. 10 is a schematic top view of a sealing sleeve according to an embodiment of the present disclosure;
FIG. 11 is a cross-sectional view of a sealing sleeve according to an embodiment of the present disclosure taken along the C-C line in FIG. 10;
FIG. 12 is a cross-sectional view of an atomizer according to an embodiment of the present disclosure taken along the D-D line in FIG. 6;
FIG. 13 is an enlarged view of an atomizer according to an embodiment of the present disclosure taken within the dashed box E in FIG. 12;
FIG. 14 is a schematic flow chart of an assembly method for an atomization core assembly according to an embodiment of the present disclosure;
FIG. 15 is an exploded schematic view of a portion of an atomizer according to an embodiment of the present disclosure;
FIG. 16 is a circuit block diagram of an atomization device according to an embodiment of the present disclosure;
FIG. 17 is a schematic flow chart of an assembly method for an atomizer according to an embodiment of the present disclosure; and
FIG. 18 is a schematic structural diagram of an assembly method for an atomizer according to an embodiment of the present disclosure.

### Reference signs:

100-atomizer, 110-atomization core, 111-atomization channel, 112-atomization portion, 113-protruding portion, 114-liquid guide member, 115-heating member, 120-porous support, 121-mounting cavity, 122-outer sidewall, 123-top end surface, 124-inner sidewall, 125-notch, 126-micropore, 127-air inlet hole, 130-sealing sleeve, 131-cylindrical portion, 1311-liquid inlet hole, 132-connecting portion, 133-surrounding portion, 1331-sleeving sub-portion, 1332-protruding sub-portion, 134-first sealing portion, 135-second sealing portion, 1351-air outlet hole, 136-insertion slot, 140-air guide tube, 141-first air guide portion, 1411-liquid passage hole, 142-second air guide portion, 1421-air guide channel, 150-base, 151-stepped recess, 152-inner wall, 153-groove, 154-blocking portion, 160-housing, 1601-bottom housing, 1602-side housing, 1603-top housing, 1604-air vent hole, 161-accommodating chamber, 162-opening, 163-e-liquid storage chamber, 180-atomization liquid, 190-circuit board assembly, 200-rod body, 300-atomization core assembly, 400-atomization device.

### DETAILED DESCRIPTION

The following, in conjunction with the accompanying drawings, provides a clear and complete description of the technical solutions in the embodiments of the present disclosure. Clearly, the described embodiments are only some of the embodiments of the present disclosure, and not all of them. All other embodiments derived by persons skilled in the art based on the embodiments of the present disclosure without creative effort are within the scope of protection of the present disclosure.

The terms "first," "second," and so on in the specification and claims of the present disclosure, as well as in the accompanying drawings, are used to distinguish between different objects, not to describe a specific order. Furthermore, the terms "including," "having," and any variations thereof, are intended to cover non-exclusive inclusions. For example, a process, method, system, product, or apparatus including a series of steps or elements is not limited to the listed steps or elements but may optionally include steps or elements not listed, or may optionally include other steps or elements inherent to such process, method, product, or apparatus.

References herein to "embodiments" or "implementations" mean that the features, structures, or characteristics described in connection with such embodiments or implementations may be included in at least one embodiment of the present disclosure. The appearance of such phrases in various places in the specification does not necessarily refer to the same embodiment, nor do they constitute independent or alternative embodiments that are mutually exclusive of other embodiments. Those skilled in the art will explicitly and implicitly understand that the embodiments described herein can be combined with other embodiments.

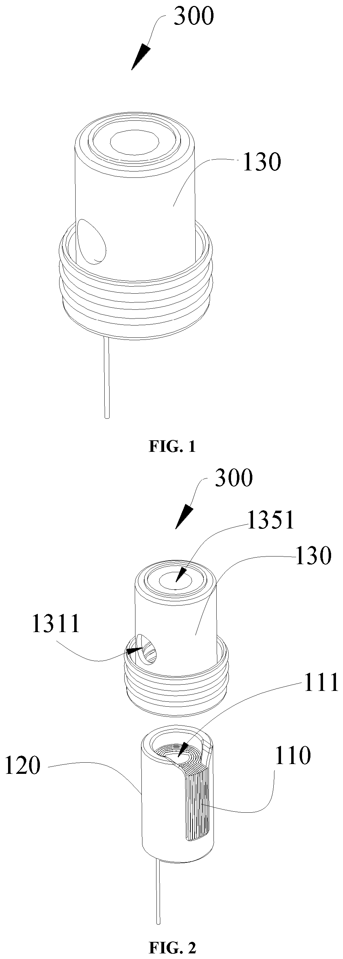

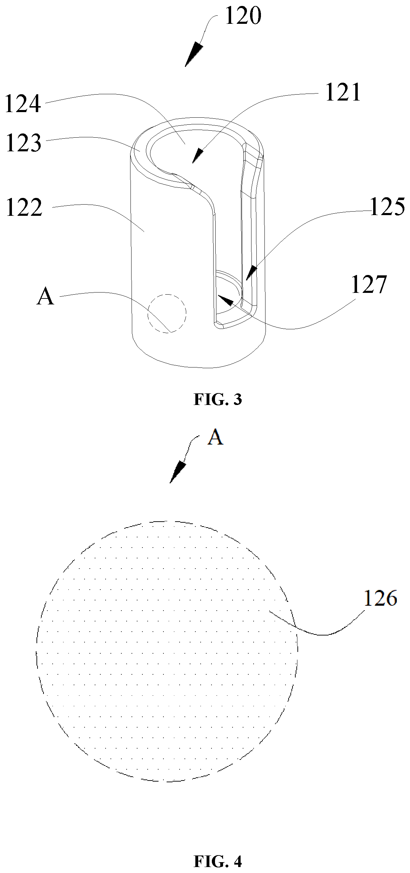

Referring to FIG. 1 to FIG. 4, the present disclosure provides an atomization core assembly 300, including: a porous support 120, an atomization core 110, and a sealing sleeve 130. The porous support 120 has a mounting cavity 121, an outer sidewall 122, and micropores 126 that allow atomization liquid to penetrate from the outer sidewall 122 into the mounting cavity 121. The atomization core 110 is disposed within the mounting cavity 121, forming a hollow atomization channel 111 therein. The sealing sleeve 130 is sleeved around the outer periphery of the porous support 120, enclosing the atomization core 110 therein. The sidewall of the sealing sleeve 130 is provided with a liquid inlet hole 1311 extending therethrough.

Preferably, the porous support 120 is disposed around the outer periphery of the atomization core 110, and may be disposed in close contact with the outer periphery of the atomization core 110, which further enhances the anti-leakage performance of the atomizer 100. Preferably, the sealing sleeve 130 is disposed around the outer periphery of the porous support 120, and may be disposed in close contact with the outer periphery of the porous support 120, which further enhances the anti-leakage performance of the atomizer 100.

It is appreciated that in the atomization core assembly 300 provided in this embodiment of the present disclosure, the atomization core 110, the porous support 120, and the sealing sleeve 130 are disposed sequentially from the inside to the outside.

Optionally, the sealing sleeve 130 may be, but is not limited to, made of silicone, rubber, or the like.

In this embodiment, the liquid inlet hole 1311 is formed through the side wall of the sealing sleeve 130, and the porous support 120 is provided with micropores 126 that allow the atomization liquid to penetrate from the outer sidewall 122 into the mounting cavity 121. When the atomization core assembly 300 is assembled to the atomizer 100, the atomization liquid 180 enters the porous support 120 from the liquid inlet hole 1311 of the sealing sleeve 130 and infiltrates the atomization core 110 through the micropores 126. The atomization core 110 heats the atomization liquid 180 and atomizes it into vapor, which then flows out of the atomization core assembly 300 through the atomization channel 111 to deliver the atomized vapor to a target object. In addition, an embodiment of the present disclosure provides a porous support 120, which is sleeved on the outer periphery of the atomization core 110. The porous support 120 is provided with a plurality of micropores 126. The plurality of micropores 126 allow the atomization liquid 180 to penetrate from the outer sidewall 122 into the mounting cavity 121, so that the atomization liquid 180 infiltrates the atomization core 110 and is atomized. Compared with the solution of using a stainless-steel support sleeved on the outer periphery of the atomization core 110, the porous support 120 provided in the present disclosure does not require an e-liquid guide hole, which is beneficial to improving the anti-leakage performance of the porous support 120, and thus improving the anti-leakage performance of the atomization core assembly 300. Furthermore, the sealing sleeve 130 is positioned around the outer periphery of the porous support 120, preventing the atomization liquid 180 from directly infiltrating the atomization core 110 through a gap between the porous support 120 and the sealing sleeve 130. This improves the leak-proof performance of the atomizer 100 and facilitates more uniform penetration of the atomization liquid 180 into the atomization core 110, enhancing the flavor of the vapor atomized by the atomization core 110 and enhancing user experience. The atomizer 100 provided in the present disclosure utilizes the sealing sleeve 130 to enhance its leak-proof performance. The sealing sleeve 130 has reduced processing and assembly costs, thereby reducing processing and assembly costs of the atomizer 100.

It is appreciated that, in the terminology of the present disclosure, "target object" refers to an object that consumes the vapor atomized from the atomization liquid 180.

It is appreciated that, in the terminology of the present disclosure, "a plurality of" refers to greater than or equal to two.

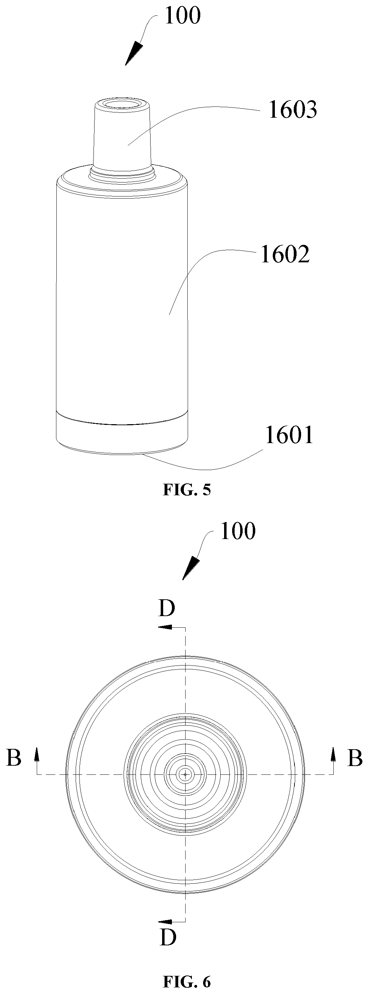

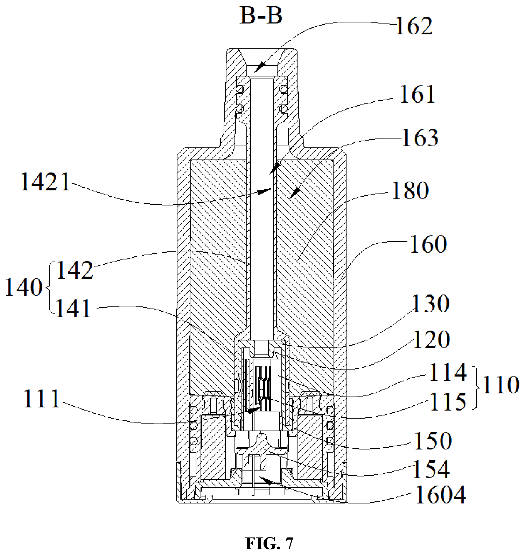

Please refer to FIG. 5 to FIG. 7. Optionally, in some embodiments, the porous support 120 further has a top end surface 123 and an inner sidewall 124 that defines the mounting cavity 121. The outer sidewall 122, the top end surface 123, and the inner sidewall 124 are connected in sequence. One end of the porous support 120 on which the sealing sleeve 130 is installed is further provided with a notch 125. The notch 125 extends through the inner sidewall 124, the top end surface 123, and the outer sidewall 122 simultaneously to communicate with the mounting cavity 121. The atomization core 110 includes a liquid guide member 114 and a heating member 115. The liquid guide member 114 at least partially extends into the notch 125. For example, the porous support 120 is generally cylindrical. The mounting cavity 121 is enclosed by the sidewalls of the porous support 120 and its shape matches the shape of the atomization core 110. For example, the mounting cavity 121 may be cylindrical, with the upper end of the mounting cavity 121 open to facilitate the insertion and removal of the atomization core 110. The outer sidewall of the atomization core 110 fits against the inner sidewall of the mounting cavity 121 to better transmit the atomization liquid 180.

The porous support 120 provided in the embodiment of the present disclosure is provided with a mounting cavity 121 and a notch 125 that extends through the inner sidewall 124, the top end surface 123 and the outer sidewall 122. In the process of mounting the porous support 120 on the periphery of the atomization core 110, the liquid guide member 114 and the heating member 115 are first stacked, where the heating member 115 is disposed on one side of the liquid guide member 114, with the liquid guide member 114 enclosing the heating member 115, and then the liquid guide member 114 and the heating member 115 are wound together. Subsequently, the liquid guide member 114 and the heating member 115 are inserted into the mounting cavity 121 of the porous support 120. A portion of the liquid guide member 114 and the heating member 115 are disposed within the mounting cavity 121. The part of the liquid guide member 114 protruding from the mounting cavity 121 is cut away along the surface of the outer sidewall 122 facing away from the inner sidewall 124, allowing at least a portion of the liquid guide member 114 to extend into the notch 125. The liquid guide member 114 positioned within the notch 125 increases its absorption capacity for the atomization liquid 180, thereby improving the atomization efficiency of the atomization core assembly 300 for the atomization liquid 180. The porous support 120 in this embodiment of the present disclosure is provided with the notch 125, facilitating manual assembly of the atomization core 110, reduces the required precision during the assembly process, and improves overall assembly efficiency.

Optionally, the liquid guide member 114 may be configured as e-liquid-conducting cotton, which is used to absorb and store the atomization liquid 180.

Optionally, in some embodiments, the heating member 115 is configured as a heating mesh, which is wound into a cylindrical heating mesh, and the outer periphery of the cylindrical heating mesh is attached to the inner circumference of the liquid guide member 114. In some other embodiments, the heating member 115 is a heating wire, which is wound into a spiral heating wire, and the outer periphery of the spiral heating wire is attached to the inner circumference of the liquid guide member 114.

Optionally, in some embodiments, the liquid guide member 114 is configured as a sheet-shaped structure, and the atomization channel 111 is formed by wrapping the sheet-shaped liquid guide member 114, with at least one end of the liquid guide member 114 located within the notch 125, and the outer periphery of the heating member 115 is attached to the inner circumference of the liquid guide member 114.

It is appreciated that at least one end of the liquid guide member 114 is located in the notch 125. Optionally, one end of the liquid guide member 114 is located in the notch 125. Optionally, both opposite ends of the liquid guide member 114 are located in the notch 125. In this embodiment, the liquid guide member 114 is configured as a sheet-shaped structure, the heating member 115 and the liquid guide member 114 are stacked, and when the atomization channel 111 is formed inside the atomization core 110, the outer peripheral surface of the heating member 115 is attached to the inner peripheral surface of the liquid guide member 114. In other words, the liquid guide member 114 is arranged on the outer periphery of the heating member 115, so that when the atomization liquid 180 infiltrates into the liquid guide member 114 through the liquid inlet hole 1311 and the micropores 126, the heating member 115 can heat and atomize the atomization liquid 180 infiltrating into the side of the liquid guide member 114 proximate to the atomization channel 111, and transport the atomized vapor out of the atomization core assembly 300 through the atomization channel 111, so as to transport the atomized vapor to the target object. At least one end of the liquid guide member 114 is located within the notch 125. Compared to a solution in which the liquid guide member 114 is positioned solely within the mounting cavity 121, in this embodiment of the present disclosure, a portion of the liquid guide member 114 is positioned within the notch 125, thus facilitating increasing the amount of atomization liquid 180 absorbed by the liquid guide member 114, facilitating sufficient atomization of the atomization liquid 180 by the heating member 115 and delivering sufficient vapor to the target object. This improves the atomization efficiency of the atomization core assembly 300 for the atomization liquid 180 and enhances the user experience.

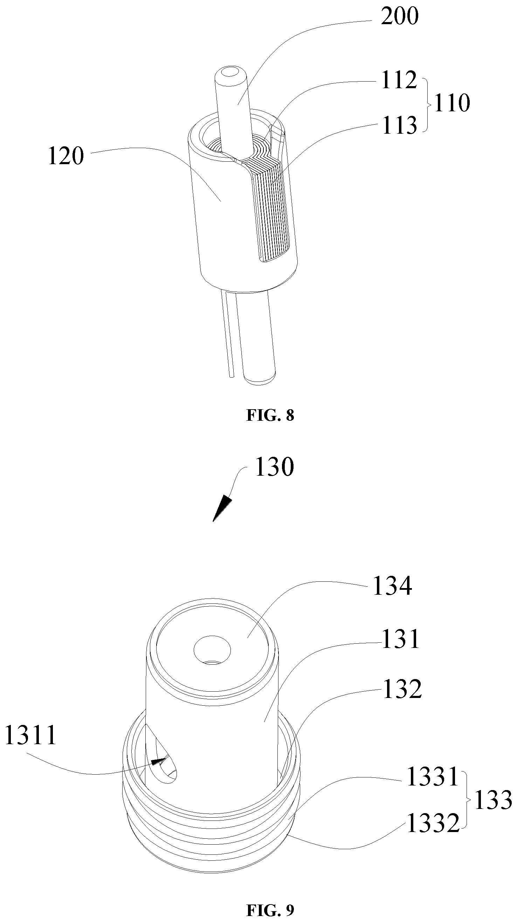

Referring to FIG. 8, the atomization core 110 optionally includes an atomization portion 112 and a protruding portion 113. The atomization portion 112 includes the atomization channel 111. The protruding portion 113 is protruding from a surface of the atomization portion 112 facing away from the atomization channel 111. The atomization portion 112 passes through the mounting cavity 121, and the protruding portion 113 passes through the notch 125.

In the embodiment of the present disclosure, when the porous support 120 is placed on the outer periphery of the atomization core 110, the liquid guide member 114 and the heating member 115 are first stacked, where the heating member 115 is disposed on one side of the liquid guide member 114, and the liquid guide member 114 encloses the heating member 115. The liquid guide member 114 and the heating member 115 are then wrapped around the outer periphery of a rod body 200, and the liquid guide member 114 and the heating member 115 are further inserted into the mounting cavity 121 of the porous support 120, forming the atomization portion 112. Furthermore, a portion of the liquid guide member 114 is disposed within the mounting cavity 121. The portion of the liquid guide member 114 protruding from the mounting cavity 121 is cut away along the surface of the outer sidewall 122 facing away from the inner sidewall 124, such that the liquid guide member 114 extending through the mounting cavity 121 forms the protruding portion 113. The protruding portion 113 increases the storage capacity of the atomization core 110 for the atomization liquid 180, thereby improving the atomization efficiency of the atomization liquid 180.

Optionally, in some embodiments, the porous support 120 is configured as a porous ceramic support.

In this embodiment, the porous support 120 is configured as a porous ceramic support. The porous ceramic support is provided with a plurality of micropores 126 that allow the atomization liquid 180 to penetrate from the outer sidewall 122 into the mounting cavity 121. The plurality of micropores 126 may be formed directly during the manufacturing process of the porous support 120, simplifying the manufacturing process of the porous support 120. Furthermore, compared to a solution in which a stainless-steel support is mounted around the outer periphery of the atomization core 110, the porous support 120 provided in the present disclosure does not require an e-liquid guide hole, thereby improving the leak-proof performance of the porous support 120 and, in turn, the leak-proof performance of the atomization core assembly 300.

Optionally, in some embodiments, the mounting cavity 121 of the porous support 120 is provided with an air inlet hole 127 on the side facing away from the notch 125, and the sealing sleeve 130 is provided with an air outlet hole 1351 on the side proximal to the notch 125. The air inlet hole 127 communicates with the air outlet hole 1351. In other words, the mounting cavity 121 of the porous support 120 is provided with an air inlet hole 127 on the side facing away from the top end surface 123, and the sealing sleeve 130 is provided with an air outlet hole 1351 on the side proximal to the top end surface 123. The air inlet hole 127 is in communication with the air outlet hole 1351.

It is appreciated that, the air inlet hole 127, the atomization channel 111, and the air outlet hole 1351 are in sequential communication.

In this embodiment, the air inlet hole 127 is configured to introduce air into the atomization core assembly 300. When the heating member 115 heats the liquid guide member 114, the atomization liquid 180 stored in the liquid guide member 114 is atomized into vapor. The vapor and air flow through the atomization channel 111 to the air outlet hole 1351 and are then transported out of the atomization core assembly 300 through the air outlet hole 1351. The air inlet hole 127 and the air outlet hole 1351 form a convective flow, which helps improve the transmission efficiency of the vapor formed by the atomization liquid 180 within the atomization core assembly 300 and enhances the smoothness of the vapor transmission. This ensures that the vapor is delivered to the target object, thereby enhancing user experience of the target object.

Optionally, orthographic projections of the liquid inlet hole 1311 and the notch 125 on the outer sidewall 122 do not overlap. In other words, the orthographic projection of the liquid inlet hole 1311 on the surface of the outer sidewall 122 facing away from the inner sidewall 124 is offset from that of the notch 125. In other words, the liquid inlet hole 1311 and the notch 125 are circumferentially offset from each other.

In this embodiment, the liquid inlet hole 1311 is offset from the notch 125, preventing the atomization liquid 180 in the e-liquid storage chamber 163 from directly entering the notch 125 through the liquid inlet hole 1311 of the sealing sleeve 130. This prevents the portion of the liquid guide member 114 located in the notch 125 from being directly infiltrated by the atomization liquid 180, which improves the infiltration uniformity of the atomization core 11by the atomization liquid 180. This avoids differences in the concentration of the atomization liquid 180 across different parts of the atomization core 110 that could affect the atomization efficiency, thereby enhancing the flavor profile of the vapor atomized by the atomization core 110 and improving the user experience of the target object.

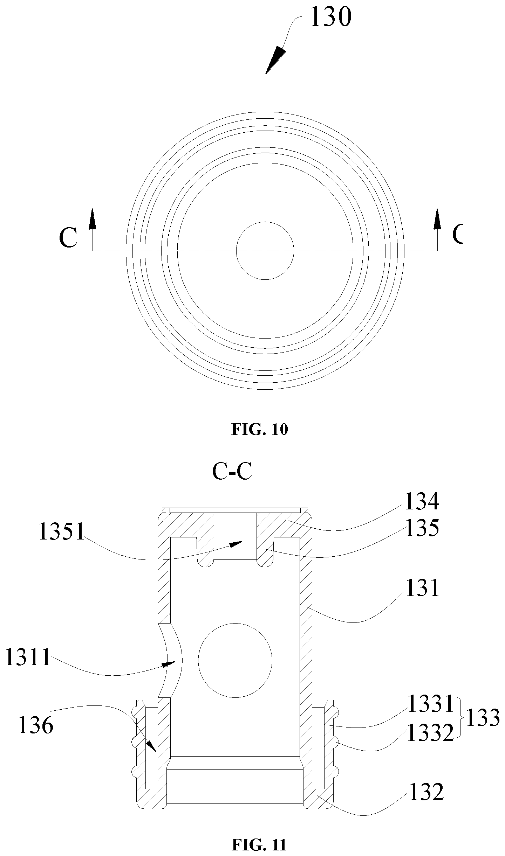

Referring to FIG. 9 to FIG. 11, in some embodiments, the sealing sleeve 130 optionally includes a cylindrical portion 131, a connecting portion 132, and a surrounding portion 133, which are sequentially connected. The cylindrical portion 131 and the surrounding portion 133 are spaced apart and disposed on the same side of the connecting portion 132. The surrounding portion 133 surrounds a part of the outer periphery of the cylindrical portion 131. The cylindrical portion 131 is sleeved around the outer periphery of the porous support 120. The cylindrical portion 131, the connecting portion 132, and the surrounding portion 133 enclose to form an insertion slot 136.

It is appreciated that the surrounding portion 133 surrounds a part of the outer periphery of the cylindrical portion 131. Alternatively, the surrounding portion 133 is sleeved over a part of the outer periphery of the cylindrical portion 131.

It is appreciated that, the connecting portion 132 connects the cylindrical portion 131 and the surrounding portion 133 at opposite ends, respectively, and the connecting portion 132 is configured to integrally connect the cylindrical portion 131 and the surrounding portion 133 into a unitary structure.

It is appreciated that, in the atomizer 100 provided in this embodiment of the present disclosure, the atomization core 110, the porous support 120, the cylindrical portion 131, and the surrounding portion 133 are arranged sequentially from the inside to the outside.

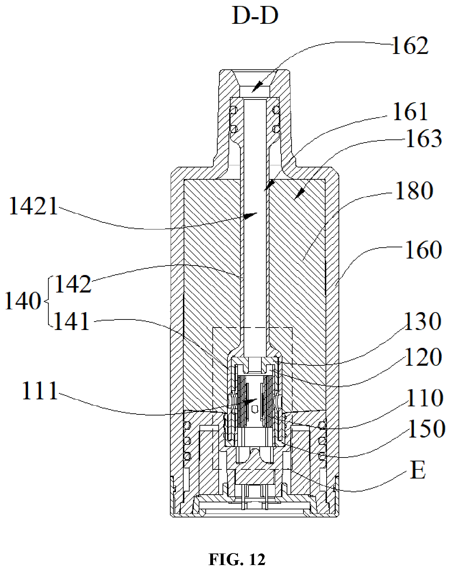

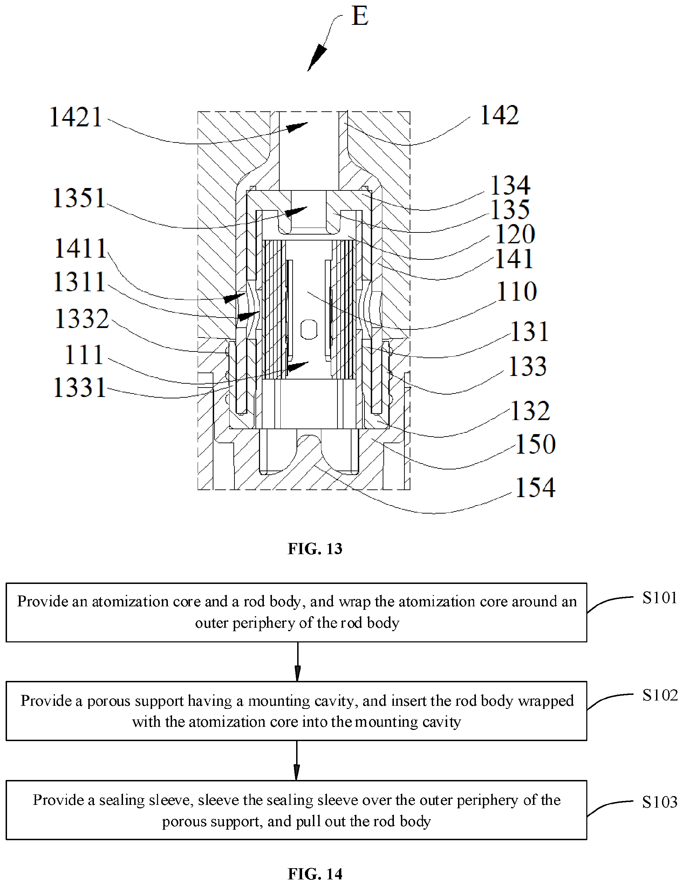

In this embodiment, the cylindrical portion 131 is sleeved on the outer periphery of the porous support 120 to prevent the atomization liquid 180 from directly entering the interior of the porous support 120 through the gap between the porous support 120 and the porous support 120, thereby preventing a part of the atomization liquid 180 from directly infiltrating the atomization core 110, thereby improving the leakage-proof performance of the atomization core assembly 300, improving the infiltration uniformity of the atomization core 110 by the atomization liquid 180, improving the flavor profile of the vapor after atomization by the atomization core 110, and improving the user experience of the target object. In addition, the cylindrical portion 131, the connecting portion 132 and the surrounding portion 133 enclose to form the insertion slot 136, which is plugged into an external component to achieve a sealed connection between the atomization core assembly 300 and the external component. When the atomization core assembly 300 is assembled into the atomizer 100, the insertion slot 136 is configured to engage with the air guide tube 140. In this case, the surrounding portion 133 is sleeved on a portion of the outer periphery of the air guide tube 140, that is, a part of the air guide tube 140 is sandwiched between the surrounding portion 133 and the cylindrical portion 131, further improving the sealing performance between the porous support 120 and the air guide tube 140, which is conducive to preventing a part of the atomization liquid 180 from entering the porous support 120 through a gap on the side of the air guide tube 140 away from the top end surface 123. Thereby, this avoids direct infiltration of the atomization liquid 180 into the atomization core 110 via a gap between the porous support 120 and the air guide tube 140, and also prevents the atomization liquid from directly entering the air guide channel 1421 through a gap on the side of the air guide tube 140 proximate to the top end surface 123. This effectively prevents the atomization liquid 180 from being directly discharged from the atomization core assembly 300 without being atomized by the atomization core 110, thus improving the leak-proof performance of the atomization core assembly 300 and enhancing the user experience of the target object. Referring to FIG. 12 and FIG. 13, optionally, when the atomization core assembly 300 is assembled into the atomizer 100, the atomizer 100 includes an air guide tube 140. The air guide tube 140 includes a first air guide portion 141 and a second air guide portion 142, which are connected to each other. The first air guide portion 141 is disposed over a portion of the outer periphery of the sealing sleeve 130. The second air guide portion 142 has an air guide channel 1421 that communicates with the atomization channel 111. The surrounding portion 133 is disposed over the outer periphery of the first air guide portion 141, and the connecting portion 132 is located on a side of the first air guide portion 141 facing away from the second air guide portion 142.

Preferably, the first air guide portion 141 is disposed over a portion of the outer periphery of the sealing sleeve 130. Alternatively, the first air guide portion 141 is fitted against a portion of the outer periphery of the sealing sleeve 130, which further enhances the leak-proof performance of the atomization core assembly 300 and the atomizer 100.

Preferably, the cylindrical portion 131 is disposed over the outer periphery of the porous support 120. Alternatively, the cylindrical portion 131 is fitted against the outer periphery of the porous support 120, thereby further improving the leak-proof performance of the atomizer 100.

Preferably, the surrounding portion 133 is disposed over the outer periphery of the first air guide portion 141. Alternatively, the surrounding portion 133 is fitted against the outer periphery of the first air guide portion 141, thereby further improving the leak-proof performance of the atomizer 100.

In this embodiment, the atomization channel 111 and the air guide channel 1421 are in fluid communication. When the atomization liquid 180 enters the atomization channel 111, the atomization core 110 heats the atomization liquid 180 and atomizes it into vapor. The atomized vapor is then transported out of the atomizer 100 through the air guide channel 1421 for delivery to the target object. In addition, the air guide channel 1421 is in communication with the atomization channel 111, and the air guide channel 1421 of the second air guide portion 142 is in communication with the atomization channel 111, which can prevent the atomized vapor from being mixed with the atomization liquid 180 again, and can also prevent non-atomized atomization liquid 180 from entering the atomization channel 111 and/or the air guide channel 1421 from mixing with the atomized vapor, thereby improving the smoothness of delivering the vapor, generated by heating the atomization liquid 180, through the air guide channel 1421 to the target object, and also improving the flavor profile of the vapor atomized by the atomization core 110. Furthermore, the surrounding portion 133 is sleeved on the outer periphery of the first air guide portion 141, that is, the first air guide portion 141 is sandwiched between the surrounding portion 133 and the cylindrical portion 131, further improving the sealing performance between the porous support 120 and the air guide tube 140, which is conducive to preventing a part of the atomization liquid 180 from entering the porous support 120 through the gap on the side of the first air guide portion 141 away from the second air guide portion 142. Thereby, this prevents a part of the atomization liquid 180 from directly infiltrating the atomization core 110 through the gap between the porous support 120 and the air guide tube 140, and also prevents the atomization liquid 180 from directly entering the air guide channel 1421 through the gap on the side of the first air guide portion 141 proximate to the second air guide portion 142. As a result, this avoids the situation where a part of the atomization liquid 180 is discharged directly through the air guide channel 1421 without being atomized by the atomization core 110. This enhances the leak-proof performance of the atomizer 100 and improves the user experience of the target object.

In some embodiments, the sealing sleeve 130 further includes a first sealing portion 134. The first sealing portion 134 is bent and connected to an end of the cylindrical portion 131 facing away from the surrounding portion 133. The first sealing portion 134 is disposed on a side of the cylindrical portion 131 proximate to the top end surface 123, and a side of the first sealing portion 134 facing the top end surface 123 abuts against the porous support 120. In other words, when the atomization core assembly 300 is assembled into the atomizer 100, the first sealing portion 134 is located on a side of the cylindrical portion 131 proximate to the second air guide portion 142, and a side of the first sealing portion 134 facing away from the second air guide portion 142 abuts against the porous support 120.

It is appreciated that, the first sealing portion 134 is bent and connected to an end of the cylindrical portion 131 facing away from the surrounding portion 133. Alternatively, the first sealing portion 134 may be bent and connected to the cylindrical portion 131, and further away from the surrounding portion 133 than the cylindrical portion 131. Alternatively, the first sealing portion 134 may be bent and connected to the cylindrical portion 131, with the first sealing portion 134, the cylindrical portion 131, and the surrounding portion 133 arranged sequentially from the inside to the outside.

In this embodiment, the first sealing portion 134 is located on the side of the cylindrical portion 131 proximate to the top end surface 123, and the first sealing portion 134 abuts against the porous support 120 on the side facing the top end surface 123. The cylindrical portion 131 and the first sealing portion 134 cooperate with each other to block the passage on the side of the porous support 120 proximate to the top end surface 123, thereby preventing the atomization liquid 180 from directly entering the atomization channel 111 through the gap between the cylindrical portion 131 and the porous support 120 as well as the gap between the first sealing portion 134 and the porous support 120, thereby improving the leakage-proof performance of the atomizer 100. On the one hand, the atomization liquid 180 that penetrates from the gap between the sealing sleeve 130 and the porous support 120 is prevented from being directly output, together with atomized vapor, from the atomizer 100 through the air guide channel 1421 without being atomized, thereby preventing the atomized vapor from mixing with the non-atomized atomization liquid 180, thereby avoiding degradation of the flavor profile produced by the atomizer 100, thereby improving the user experience of the target object. On the other hand, the atomization liquid 180 that penetrates from the gap between the sealing sleeve 130 and the porous support 120 is prevented from dripping onto the atomization core 110. Thereby, this avoids differences in the concentration of the atomization liquid 180 across different parts of the atomization core 110, improves the atomization efficiency of the atomization liquid 180 by the atomization core 110, and consequently enhances the flavor profile of the vapor atomized by the atomization core 110, ultimately improving the user experience of the target object.

In some embodiments, the sealing sleeve 130 further includes a second sealing portion 135. The second sealing portion 135 connects to an end of the first sealing portion 134 facing away from the cylindrical portion 131. The second sealing portion 135 and the cylindrical portion 131 are located on the same side of the first sealing portion 134. A side of the second sealing portion 135 facing the cylindrical portion 131 abuts against the porous support 120. The second sealing portion 135 has an air outlet hole 1351 that communicates the atomization channel 111 with the air guide channel 1421.

It is appreciated that the second sealing portion 135 connects to an end of the first sealing portion 134 facing away from the cylindrical portion 131. Alternatively, the two opposite ends of the first sealing portion 134 are respectively connected to the second sealing portion 135 and the cylindrical portion 131.

It is appreciated that the second sealing portion 135 and the cylindrical portion 131 are disposed on the same side of the first sealing portion 134. Alternatively, an end of the second sealing portion 135 facing away from the first sealing portion 134 is positioned toward the atomization channel 111.

It is appreciated that the atomization channel 111, the air outlet hole 1351, and the air guide channel 1421 are sequentially communicated, allowing vapor atomized by the atomization core 110 to exit the atomizer 100 through the air outlet hole 1351 and the air guide channel 1421.

In this embodiment, the second sealing portion 135 and the cylindrical portion 131 are located on the same side of the first sealing portion 134, and an end of the second sealing portion 135 facing away from the first sealing portion 134 is disposed toward the atomization channel 111, and a side of the second sealing portion 135 facing the cylindrical portion 131 abuts against the porous support 120, so that the second sealing portion 135 blocks the passage between the side of the porous support 120 facing the atomization channel 111 and the side of the second sealing portion 135 facing the cylindrical portion 131, thereby preventing the atomization liquid 180 from entering the atomization channel 111 through the gap between the second sealing portion 135 and the porous support 120, thereby improving the leakage-proof performance of the atomization core assembly 300. On the one hand, this prevents the atomization liquid 180 that infiltrates through the gap between the sealing sleeve 130 and the porous support 120 from being directly output, together with the already atomized vapor, through the air guide channel 1421 without being atomized, thereby preventing the atomized vapor from mixing with the non-atomized atomization liquid 180 and degrading the flavor profile of the atomizer 100, so as to enhance the user experience of the target object. On the other hand, this prevents the atomization liquid 180 that infiltrates through the gap between the sealing sleeve 130 and the porous support 120 from dripping onto the atomization core 110, thereby avoiding differences in the concentration of the atomization liquid 180 among different parts of the atomization core 110. This improves the atomization effect of the atomization liquid 180 by the atomization core 110, further enhancing the flavor profile of the vapor atomized by the atomization core 110 and improving the user experience of the target object.

Referring to FIG. 14, the present disclosure further provides an assembly method for an atomization core assembly 300, including:
S101: Provide an atomization core 110 and a rod body 200, and wrap the atomization core 110 around an outer periphery of the rod body 200.

Specifically, the atomization core 110 includes a liquid guide member 114 and a heating member 115, where the liquid guide member 114 is a sheet-shaped structure. The liquid guide member 114 and the heating member 115 are first stacked together, where the heating member 115 is disposed on one side of the liquid guide member 114, and the liquid guide member 114 encloses the heating member 115. The liquid guide member 114 and the heating member 115 are then wrapped around an outer periphery of the rod body 200, such that an outer circumferential surface of the heating member 115 is attached to an inner circumferential surface of the liquid guide member 114.

S102: Provide a porous support 120 having a mounting cavity 121, and insert the rod body 200 wrapped with the atomization core 110 into the mounting cavity 121.

Specifically, the liquid guide member 114 and the heating member 115 are inserted into the mounting cavity 121 of the porous support 120, so that a portion of the liquid guide member 114 and the heating member 115 are positioned within the mounting cavity 121.

S103: Provide a sealing sleeve 130, sleeve the sealing sleeve 130 over the outer periphery of the porous support 120, and pull out the rod body 200.

Specifically, the sealing sleeve 130 includes a cylindrical portion 131, a connecting portion 132, and a surrounding portion 133 connected in sequence, the cylindrical portion 131 and the surrounding portion 133 are spaced apart and arranged on the same side of the connecting portion 132, the surrounding portion 133 is arranged around a part of the outer periphery of the cylindrical portion 131, and the cylindrical portion 131 is sleeved on the outer periphery of the porous support 120. The sealing sleeve 130 further includes a first sealing portion 134, the first sealing portion 134 is bent and connected to an end of the cylindrical portion 131 away from the surrounding portion 133, and the first sealing portion 134 is located on a side of the cylindrical portion 131 proximate to the top end surface 123. A side of the first sealing portion 134 facing the top end surface 123 abuts against the porous support 120. The sealing sleeve 130 further includes a second sealing portion 135, which connects to an end of the first sealing portion 134 facing away from the cylindrical portion 131. The second sealing portion 135 and the cylindrical portion 131 are located on the same side of the first sealing portion 134. A side of the second sealing portion 135 facing the cylindrical portion 131 abuts against the porous support 120, thereby achieving a sealed connection between the sealing sleeve 130 and the porous support 120 and enhancing the leak-proof performance of the atomization core assembly 300.

Optionally, in some embodiments, after the sealing sleeve 130 is sleeved around the outer periphery of the porous support 120, the rod body 200 is removed. In some other embodiments, when the sealing sleeve 130 is sleeved around the porous support 120, the rod body 200 remains in place. When the atomization core assembly 300 is assembled into the atomizer 100, the rod body 200 is removed after the base 150 is assembled with the atomizer 100.

In the assembly method for the atomization core assembly 300 provided in this embodiment, the porous support 120 is provided with a mounting cavity 121, which facilitates an operator to place the atomization core 110 into the mounting cavity 121. Furthermore, the assembly method provides a sealing sleeve 130, which is beneficial for improving the sealed connection between the sealing sleeve 130 and the porous support 120, enhancing the leak-proof performance of the atomization core assembly 300. The assembly method is simple, which facilitates operation by the operator.

Optionally, in some embodiments, the atomization core 110 includes a liquid guide member 114, which is configured as a sheet-shaped structure. Wrapping the atomization core 110 around an outer periphery of the rod body 200 includes winding the sheet-shaped liquid guide member 114 around the outer periphery of the rod body 200, such that two ends of the sheet-shaped liquid guide member 114 are attached to each other.

In this embodiment, the liquid guide member 114 is configured as a sheet-shaped member. The liquid guide member 114 and the heating member 115 are first stacked, with the heating member 115 positioned on a side of the liquid guide member 114. The liquid guide member 114 then wraps the heating member 115 therein. The liquid guide member 114 and the heating member 115 are then wrapped around the outer periphery of the rod body 200, such that the outer periphery of the heating member 115 is attached to the inner circumference of the liquid guide member 114. The liquid guide member 114 and the heating member 115 are wrapped around the outer periphery of the rod body 200. The rod body 200 provides support for the wrapped structure, facilitating placement of the liquid guide member 114 and the heating member 115 within the mounting cavity 121. The two ends of the sheet-shaped liquid guide member 114 are attached to each other, making it easier for an operator to hold and insert the atomization core 110 into the mounting cavity 121 and facilitating further assembly of the atomization core 110.

Optionally, in some embodiments, the porous support 120 is provided with a notch 125. Inserting the rod body 200 wrapped with the atomization core 110 into the mounting cavity 121 includes: aligning the two ends of the sheet-shaped liquid guide member 114 with the notch 125 and inserting the sheet-shaped liquid guide member 114 into the mounting cavity 121; and cutting off a portion of an end portion of the sheet-shaped liquid guide member 114 that extends out of the notch 125.

Optionally, in some embodiments, one end portion of the sheet-shaped liquid guide member 114 extends out of the notch 125, and a portion of the end portion of the liquid guide member 114 extending out of the notch 125 is cut away. In some other embodiments, both opposite ends of the sheet-shaped liquid guide member 114 extend out of the notch 125, and the portion of the end portion of the liquid guide member 114 extending out of the notch 125 is cut away.

In the assembly method for the atomization core assembly 300 provided in this embodiment, the sheet-shaped liquid guide member 114 and the heating member 115 are disposed around the outer periphery of the rod body 200 and inserted into the mounting cavity 121, such that a portion of the liquid guide member 114 and the heating member 115 are disposed within the mounting cavity 121. Furthermore, the two ends of the sheet-shaped liquid guide member 114 are attached to each other so as to be inserted into the notch 125, leaving a portion extending out of the notch 125. The portion of the end portion of the sheet-shaped liquid guide member 114 extending out of the notch 125 is cut away so that the end portion of the liquid guide member 114 facing away from the mounting cavity 121 is flush with the outer sidewall 122, thereby preventing interference with the further assembly of the porous support 120 and the sealing sleeve 130. Furthermore, the portion of the liquid guide member 114 located within the notch 125 can enhance the e-liquid storage capacity of the atomization core 110, thereby improving the atomization efficiency of the atomization core assembly 300 for the atomization liquid 180.

Optionally, in some embodiments, the porous support 120 is configured as a porous ceramic support provided with a plurality of micropores 126 that allow the atomization liquid 180 to penetrate from the outer sidewall 122 into the mounting cavity 121.

In this embodiment, the porous support 120 is configured as a porous ceramic support provided with a plurality of micropores 126 that allow the atomization liquid 180 to penetrate from the outer sidewall 122 into the mounting cavity 121. These micropores 126 may be formed directly during the manufacturing process of the porous support 120, simplifying its manufacturing process. Furthermore, compared to a solution where a stainless-steel support is sleeved around the outer periphery of the atomization core 110, the porous support 120 provided in the present disclosure does not require an e-liquid guide hole, which improves the leak-resistant performance of the porous support 120 and subsequently improves the leak-resistant performance of the atomization core assembly 300.

Optionally, in the assembly method for the atomization core assembly 300 provided in an embodiment of the present disclosure, the sealing sleeve 130 is provided with a liquid inlet hole 1311 extending therethrough on its side wall, and the porous support 120 is provided with a notch 125. Sleeving the sealing sleeve 130 over the outer periphery of the porous support 120 includes that orthographic projections of the liquid inlet hole 1311 and the notch 125 on the outer sidewall 122 do not overlap.

In the assembly method for the atomization core assembly 300 provided in this embodiment, the orthographic projections of the liquid inlet hole 1311 and the notch 125 on the outer sidewall 122 do not overlap. In other words, the liquid inlet hole 1311 is positioned offset from the notch 125, preventing the atomization liquid 180 within the e-liquid storage chamber 163 from directly entering the notch 125 through the liquid inlet hole 1311 of the sealing sleeve 130. This prevents the portion of the liquid guide member 114 located in the notch 125 from being directly infiltrated by the atomization liquid 180, which improves the infiltration uniformity of the atomization core 110 by the atomization liquid 180. This avoids differences in the concentration of the atomization liquid 180 across different parts of the atomization core 110 that could affect the atomization efficiency, thereby enhancing the flavor profile of the vapor atomized by the atomization core 110 and improving the user experience of the target object.

Referring to FIG. 5 to FIG. 7, the present disclosure further provides an atomizer 100 including the atomization core assembly 300 provided in this embodiment of the present disclosure.

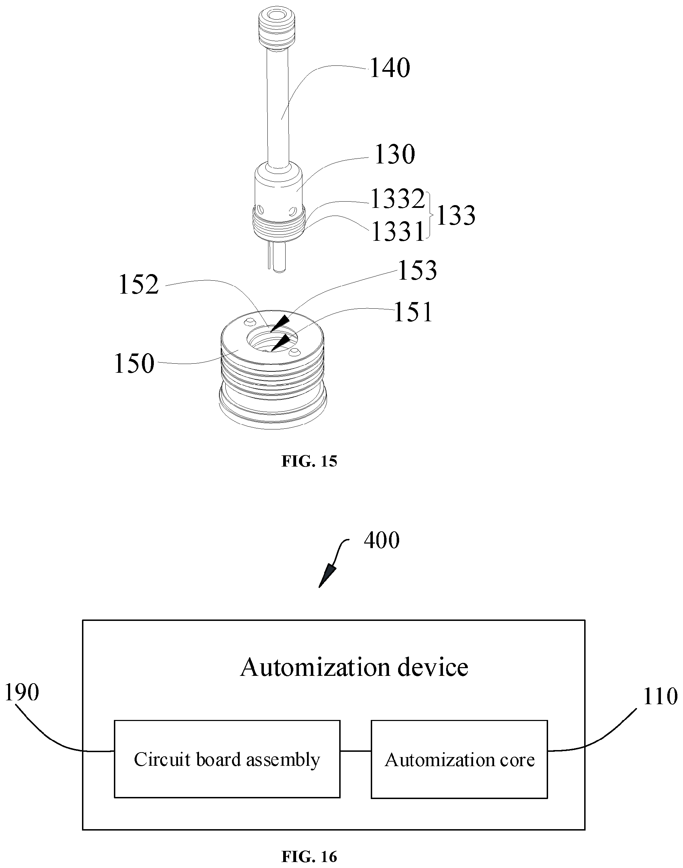

In this embodiment, the atomization core assembly 300 exhibits improved leak-resistant performance. Consequently, when the atomization core assembly 300 is assembled into the atomizer 100, the atomizer 100 also demonstrates enhanced leak-resistant performance. Additionally, the atomization liquid 180 infiltrates the atomization core 110 with a high degree of uniformity, which contributes to improving the flavor profile of the vapor produced by the atomization core 110 and ultimately enhances the user experience of the target object. Referring to FIG. 15, in some embodiments, the atomizer 100 further includes a base 150 and an air guide tube 140. The air guide tube 140 is mounted around a portion of the outer periphery of the sealing sleeve 130. The base 150 is provided with a stepped recess 151. The sealing sleeve 130 of the atomization core assembly 300 is at least partially located within the stepped recess 151. The sealing sleeve 130 is configured to achieve a sealed connection between the base 150 and the air guide tube 140. In other words, the base 150 is provided with a stepped recess 151 and an inner wall 152 defining the stepped recess 151. The surrounding portion 133 is inserted into the stepped recess 151 and abuts against the inner wall 152 of the stepped recess 151.

In this embodiment, when the atomization liquid 180 enters the atomization channel 111, the atomization core 110 heats the atomization liquid 180 and atomizes it into vapor. The atomized vapor is transported out of the atomizer 100 through the air guide channel 1421 to deliver the atomized vapor to the target object. The sealing sleeve 130 of the atomization core assembly 300 is at least partially located in the stepped recess 151. The sealing sleeve 130 is configured to achieve a sealed connection between the base 150 and the air guide tube 140, which can prevent the atomization liquid 180 from leaking out of the gap between the base 150 and the atomization core assembly 300, thereby preventing the atomizer 100 from leaking. This improves the anti-leakage effect of the atomizer 100 and also improves the utilization rate of the atomization liquid 180, which is beneficial to improving the user experience of the target object. Furthermore, the surrounding portion 133 is inserted into the stepped recess 151, and the sealing sleeve 130, the air guide tube 140, the porous support 120, and the atomization core 110 are all inserted into the stepped recess 151 of the base 150, so as to fix the sealing sleeve 130, the air guide tube 140, the porous support 120, and the atomization core 110 on the base 150. The surrounding portion 133 is inserted into the stepped recess 151 and abuts against the inner wall 152 of the stepped recess 151. The opposite ends of the surrounding portion 133 respectively abut against the inner wall 152 of the stepped recess 151 of the base 150 and the air guide tube 140. A surface of the surrounding portion 133 facing the stepped recess 151 is in close contact with the inner wall 152 of the stepped recess 151, preventing the atomization liquid 180 from leaking through the gap between the base 150 and the surrounding portion 133, thereby preventing leakage of the atomizer 100. This improves the anti-leakage effect of the atomizer 100 and increases the utilization rate of the atomization liquid 180, thereby enhancing the user experience of the target object.

In some embodiments, the base 150 is further provided with a groove 153, which is located on the inner wall 152 and encircles the inner circumference of the stepped recess 151. The surrounding portion 133 includes a sleeve sub-section 1331 and a protruding sub-section 1332. The sleeve sub-section 1331 sleeves around the outer periphery of the first air guide portion 141, while the protruding sub-section 1332 protrudes from the sleeve sub-section 1331 on a surface facing away from the first air guide portion 141. The protruding sub-section 1332 surrounds the sleeve sub-section 1331 and is located within the groove 153.

It is appreciated that the sleeve sub-section 1331 is sleeved around the outer periphery of the first air guide portion 141. Alternatively, the sleeve sub-section 1331 is attached to the outer periphery of the first air guide portion 141.

It is appreciated that, that the protruding sub-section 1332 is provided on a surface of the sleeve sub-section 1331 that faces away from the first air guide portion 141 may mean that the protruding sub-section 1332 is provided farther away from the first air guide portion 141 than the sleeve sub-section 1331.

It is appreciated that, that the protruding sub-section 1332 is provided around the sleeve sub-section 1331 may mean that the protruding sub-section 1332 is provided around the outer periphery of the sleeve sub-section 1331.

In this embodiment, the sleeve sub-section 1331 is provided around the outer periphery of the first air guide portion 141 to achieve sealing between the first air guide portion 141 and the porous support 120, preventing the atomization liquid 180 from entering the porous support 120 through the gap between the first air guide portion 141 and the surrounding portion 133, as well as the gap between the first air guide portion 141 and the cylindrical portion 131, thereby improving the anti-leakage effect of the atomizer 100. In addition, a side of the sleeve sub-section 1331 facing away from the first air guide portion 141 abuts against the stepped recess 151, achieving a sealed connection between the sealing sleeve 130 and the base 150, preventing the atomization liquid 180 from leaking out of the gap between the base 150 and the surrounding portion 133, avoiding leakage of the atomizer 100, and improving the anti-leakage effect of the atomizer 100. Furthermore, the protruding sub-section 1332 protrudes from the surface of the sleeve sub-section 1331 facing away from the first air guide portion 141, and the protruding sub-section 1332 is located in the groove 153 encircling the stepped recess 151. By embedding the protruding sub-section 1332 in the groove 153, the sealing performance between the sealing sleeve 130 and the base 150 can be further improved, further preventing the atomization liquid 180 from leaking out of the gap between the base 150 and the protruding sub-section 1332, greatly improving the anti-leakage performance of the atomizer 100.

In some embodiments, the atomizer 100 further includes a housing 160, which includes a bottom housing 1601. The housing 160 is sleeved around the outer periphery of the base 150 and the air guide tube 140. The bottom housing 1601 is disposed on one side of the base 150 and the air guide tube 140. The bottom housing 1601 is provided with an air vent hole 1604. The base 150 further includes a blocking portion 154. The blocking portion 154 is disposed on a surface of the stepped recess 151 facing the atomization core assembly 300, and the orthographic projection of the air vent hole 1604 on the surface of the stepped recess 151 facing the atomization core assembly 300 falls within the range of the blocking portion 154. The blocking portion 154 is configured to prevent liquid from dripping into the air vent hole 1604.

In this embodiment, the housing 160 is sleeved around the base 150 and the air guide tube 140 to protect the base 150 and the air guide tube 140. The bottom housing 1601 is disposed on one side of the base 150 and the air guide tube 140. When the atomizer 100 is in use, the bottom housing 1601 is disposed below the base 150 and the air guide tube 140. The bottom housing 1601 is provided with an air vent hole 1604 for allowing air to enter the atomizer 100. When the orthographic projection of the air vent hole 1604 on the surface of the stepped recess 151 facing the atomization core assembly 300 falls within the range of the blocking portion 154 and the atomization liquid 180 is atomized by the atomization core 110 to form vapor, some of the vapor may re-condense into liquid. The re-condensed liquid, along with some of the atomization liquid 180, may drip through the air inlet hole 127 of the porous support 120 and into the blocking portion 154. The blocking portion 154 blocks the re-condensed liquid and some of the atomization liquid 180, preventing them from dripping directly into the air vent hole 1604 and affecting the use of the atomizer 100, thereby improving the user experience of the target object.

Optionally, the housing 160 further includes a side housing 1602 and a top housing 1603. The bottom housing 1601, the side housing 1602, and the top housing 1603 are sequentially connected. The bottom housing 1601, the side housing 1602, and the top housing 1603, together with the base 150, enclose to form an accommodating chamber 161. The top housing 1603 is provided with an opening 162 communicating with the accommodating chamber 161. A portion of the air guide tube 140 is located in the accommodating chamber 161 and a portion of the air guide tube 140 communicates with the opening 162. The housing 160 and the air guide tube 140 are spaced apart and enclose to form an e-liquid storage chamber 163 for setting the atomization liquid 180. The first air guide portion 141 is provided with a liquid passage hole 1411, and the sealing sleeve 130 is provided with a liquid inlet hole 1311. The e-liquid storage chamber 163, the liquid passage hole 1411, and the liquid inlet hole 1311 are sequentially communicated.

It is appreciated that the atomization channel 111, the air outlet hole 1351 of the second sealing portion 135 of the sealing sleeve 130, the air guide channel 1421, and the opening 162 are sequentially communicated to enable the vapor atomized by the atomization core 110 to be transported out of the atomizer 100 and delivered to the target object.

It is appreciated that the accommodating chamber 161 includes the e-liquid storage chamber 163 and a chamber for setting the air guide tube 140.

Optionally, the atomization liquid 180 may be, but is not limited to, e-liquid, liquid healthcare medication, or liquid therapeutic medication.

In this embodiment, the housing 160 and the air guide tube 140 are spaced apart and enclose to form the e-liquid storage chamber 163. The e-liquid storage chamber 163 is configured to set the atomization liquid 180. The atomization liquid 180 enters the atomization channel 111 through the liquid passage hole 1411 of the first air guide portion 141, the liquid inlet hole 1311 of the sealing sleeve 130, and the micropores of the porous support 120 in sequence to infiltrate the atomization core 110. The atomization core 110 heats the stored atomization liquid 180 and atomizes it into vapor. The atomized vapor is delivered out of the atomizer 100 through the air outlet hole 1351, the air guide channel 1421, and the opening 162, so as to transport the atomized vapor to the target object. The e-liquid storage chamber 163, the liquid passage hole 1411, and the liquid inlet hole 1311 are communicated in sequence, which is conducive to controlling the speed of the atomization liquid 180 entering the atomization channel 111 from the e-liquid storage chamber 163, enhancing the infiltration uniformity of the atomization core 110 by the atomization liquid 180, improving the flavor profile of the vapor atomized by the atomization core 110, and elevating the user experience of the target object.

Optionally, in some embodiments, the orthogonal projection of the liquid passage hole 1411 on the surface of the sealing sleeve 130 facing the air guide tube 140 falls within the range of the liquid inlet hole 1311. In other words, the diameter of the liquid passage hole 1411 is smaller than the diameter of the liquid inlet hole 1311.

In this embodiment, since the orthogonal projection of the liquid passage hole 1411 on the surface of the sealing sleeve 130 facing the air guide tube 140 falls within the range of the liquid inlet hole 1311, it is beneficial for the atomization liquid 180 to enter the liquid inlet hole 1311 from the e-liquid storage chamber 163 via the liquid passage hole 1411. This prevents a portion of the atomization liquid 180 from infiltrating into the gap between the first air guide portion 141 and the sealing sleeve 130 due to an excessively small diameter of the liquid inlet hole 1311, thereby improving the anti-leakage effect of the atomizer 100. Furthermore, this also facilitates a uniform entry of the atomization liquid 180 into the atomization channel 111 from the liquid passage hole 1411, the liquid inlet hole 1311, and the micropores of the porous support 120. This improves the flavor profile of the vapor atomized by the atomization core 110 and enhances the user experience for the target object. Optionally, in some embodiments, a plurality of liquid passage holes 1411 are provided, and a plurality of liquid inlet holes 1311 are also provided. The number of liquid passage holes 1411 may be two, three, four, or more. The number of liquid inlet holes 1311 may be two, three, four, or more.

In this embodiment, a plurality of liquid passage holes 1411 and a plurality of liquid inlet holes 1311 are provided. The plurality of liquid passage holes 1411 and the plurality of liquid inlet holes 1311 facilitate accelerated flow of the atomization liquid 180 from the e-liquid storage chamber 163 into the atomization channel 111. This enables thorough infiltration of the atomization core 110 by the atomization liquid 180, thereby improving the infiltration uniformity of the atomization core 110 by the atomization liquid 180, improving the flavor profile of the vapor atomized by the atomization core 110, and elevating the user experience of the target user.

Please refer to FIG. 16. The present disclosure further provides an atomization device 400, which includes the atomizer 100 provided by the present disclosure.

In this embodiment, the atomizer 100 exhibits improved leak-resistant performance, so that when the atomizer 100 is used in the atomization device 400, the atomization device 400 also exhibits improved leak-resistant performance. In addition, in the atomization device 400, the atomization liquid 180 exhibits high uniformity in infiltrating the atomization core 110, which helps to improve the flavor profile of the vapor atomized by the atomization core 110 and enhances the user experience for the target object.

In some embodiments, the atomization device 400 further includes a circuit board assembly 190. The circuit board assembly 190 is disposed on a side of the base 150 facing away from the e-liquid storage chamber 163, and the circuit board assembly 190 is electrically connected to the atomization core 110.

It is appreciated that the circuit board assembly 190 is electrically connected to the heating member 115 of the atomization core 110, so that the heating member 115 heats and atomizes the atomization liquid 180 stored in the liquid guide member 114.

In this embodiment, the circuit board assembly 190 supplies electrical power to the atomization core 110. This enables the atomization core 110 to heat up when the atomization liquid 180 infiltrates and is stored in the atomization core 110, thereby atomizing the atomization liquid 180. The vapor atomized by the atomization core 110 is then delivered out of the atomizer 100 through the air guide channel 1421 to the target object.

Optionally, in some embodiments, the circuit board assembly 190 and the atomizer 100 share the housing 160 and are assembled into an integrated structure. In some other embodiments, the circuit board assembly 190 and the atomizer 100 are separate structures and can be detachably connected.

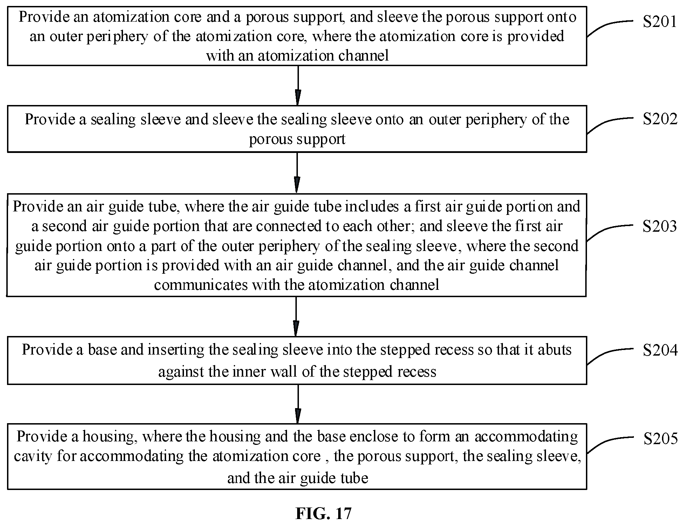

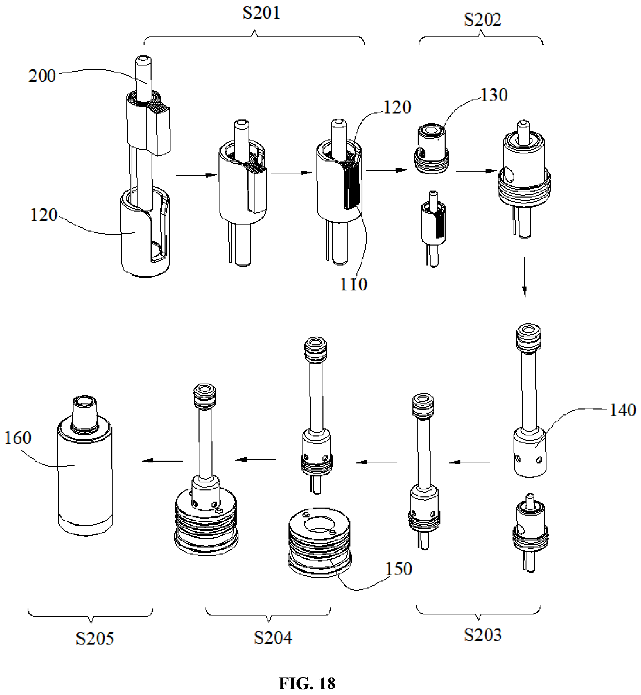

Please refer to FIG. 17 and FIG. 18. Optionally, the present disclosure further provides an assembly method for an atomizer 100, which includes the following steps:
S201: Provide an atomization core 110 and a porous support 120, and sleeve the porous support 120 onto an outer periphery of the atomization core 110, where the atomization core 110 is provided with an atomization channel 111.

Specifically, the atomization core 110 includes a liquid guide member 114 and a heating member 115. First, the liquid guide member 114 and the heating member 115 are stacked, with the heating member 115 located on one side of the liquid guide member 114, and the liquid guide member 114 encloses the heating member 115. Then, the liquid guide member 114 and the heating member 115 are wound around an outer periphery of a rod body 200. Furthermore, the liquid guide member 114 and the heating member 115 are inserted into a mounting cavity 121 of the porous support 120. A portion of the liquid guide member 114 that is disposed within the mounting cavity 121 and the heating member 115 form an atomization portion 112. Furthermore, a portion of the liquid guide member 114 is disposed in the mounting cavity 121, and a portion of the liquid guide member 114 protruding from the mounting cavity 121 is cut along a surface of an outer sidewall 122 facing away from an inner sidewall 124, so that the liquid guide member 114 disposed within the mounting cavity 121 forms a protruding portion 113. The atomization portion 112 and the protruding portion 113 form the atomization core 110, which is provided with the atomization channel 111. S202: Provide a sealing sleeve 130 and sleeve the sealing sleeve 130 onto an outer periphery of the porous support 120.

Specifically, the sealing sleeve 130 includes a cylindrical portion 131, a connecting portion 132, and a surrounding portion 133 that are sequentially connected. The cylindrical portion 131 and the surrounding portion 133 are spaced apart and disposed on the same side of the connecting portion 132. The surrounding portion 133 surrounds a part of an outer periphery of the cylindrical portion 131. The cylindrical portion 131 is sleeved around the outer periphery of the porous support 120. The sealing sleeve 130 further includes a first sealing portion 134. The first sealing portion 134 is bent and connected to an end of the cylindrical portion 131 facing away from the surrounding portion 133. The first sealing portion 134 is disposed on a side of the cylindrical portion 131 proximate to a second air guide portion 142, and a side of the first sealing portion 134 facing away from the second air guide portion 142 abuts against the porous support 120. The sealing sleeve 130 further includes a second sealing portion 135, which connects to an end of the first sealing portion 134 facing away from the cylindrical portion 131, and the second sealing portion 135 and the cylindrical portion 131 are located on the same side of the first sealing portion 134. A side of the second sealing portion 135 facing the cylindrical portion 131 abuts against the porous support 120 to achieve a sealed connection between the sealing sleeve 130 and the porous support 120, thereby enhancing the anti-leakage effect of the atomizer 100.

Specifically, the sealing sleeve 130 is provided with a liquid inlet hole 1311. An orthogonal projection of the liquid inlet hole 1311 on a surface of the outer sidewall 122 facing away from the inner side wall 124 is offset from that of the notch 125.

S203: Provide an air guide tube 140, where the air guide tube 140 includes a first air guide portion 141 and a second air guide portion 142 that are connected to each other; and sleeve the first air guide portion 141 onto a part of the outer periphery of the sealing sleeve 130, where the second air guide portion 142 is provided with an air guide channel 1421, and the air guide channel 1421 communicates with the atomization channel 111.

Specifically, the first air guide portion 141 is sleeved on the outer periphery of the cylindrical portion 131. The surrounding portion 133 is sleeved on the outer periphery of the first air guide portion 141. The connecting portion 132 is located on a side of the first air guide portion 141 facing away from the second air guide portion 142. In addition, the second sealing portion 135 is provided with an air outlet hole 1351, and the air outlet hole 1351 communicates with the atomization channel 111 and the air guide channel 1421. Specifically, the first air guide portion 141 is provided with a liquid passage hole 1411, and the liquid passage hole 1411 communicates with the liquid inlet hole 1311.

S204: Provide a base 150 and insert the sealing sleeve 130 into the stepped recess 151 so that it abuts against the inner wall 152 of the stepped recess 151.

Specifically, the base 150 is provided with a stepped recess 151 and an inner wall 152 that defines the stepped recess 151. The surrounding portion 133 of the sealing sleeve 130 is inserted into the stepped recess 151 and abuts against the inner wall 152 of the stepped recess 151 to fix the atomization core 110, the porous support 120, the sealing sleeve 130, and the air guide tube 140 onto the base 150.

S205: Provide a housing 160, where the housing 160 and the base 150 enclose to form an accommodating cavity 161 for accommodating the atomization core 110, the porous support 120, the sealing sleeve 130, and the air guide tube 140.

Specifically, the housing 160 is further provided with an opening 162 that communicates with the accommodating cavity 161. A part of the air guide tube 140 is located in the accommodating cavity 161, and a part thereof communicates with the opening 162. The housing 160 and the air guide tube 140 are spaced apart and enclose to form an e-liquid storage chamber 163, which is configured for setting the atomization liquid 180. The first air guide portion 141 is provided with a liquid passage hole 1411, and the sealing sleeve 130 is provided with a liquid inlet hole 1311. The e-liquid storage chamber 163, the liquid passage hole 1411, and the liquid inlet hole 1311 are sequentially in communication. Finally, the rod body 200 is pulled out.

Specifically, the e-liquid storage chamber 163, the liquid passage hole 1411, and the liquid inlet hole 1311 are sequentially in communication.

In the assembly method for the atomizer 100 provided in the present disclosure, the atomization core 110 is provided with the notch 125, which facilitates the assembly of the atomization core 110. Furthermore, the assembly manner of sleeving the sealing sleeve 130 onto the outer periphery of the porous support 120 is simple and can improve the anti-leakage effect of the atomizer 100.

In the present disclosure, the terms "embodiment" and "implementation" mean that a specific feature, structure, or characteristic described in connection with the embodiment may be included in at least one embodiment of the present disclosure. The appearance of the phrases in various places in the specification does not necessarily all refer to the same embodiment, nor are they independent or alternative embodiments that are mutually exclusive of other embodiments. Those skilled in the art will explicitly and implicitly understand that the embodiments described in the present disclosure can be combined with other embodiments. Furthermore, it should also be understood that the features, structures, or characteristics described in the various embodiments of the present disclosure can be combined arbitrarily, as long as there is no contradiction between them, to form another embodiment that does not depart from the spirit and scope of the technical solution of the present disclosure.

Finally, it should be noted that the above embodiments are only intended to illustrate the technical solutions of the present disclosure and not to limit them. Although the present disclosure has been described in detail with reference to the preferred embodiments, those of ordinary skill in the art should understand that modifications or equivalent substitutions can be made to the technical solutions of the present disclosure without departing from the spirit and scope of the technical solutions of the present disclosure.

## Claims

1. An atomization core assembly, wherein the atomization core assembly comprises:
a porous support, wherein the porous support is provided with a mounting cavity, an outer sidewall, and micropores configured to allow atomization liquid to permeate from the outer sidewall into the mounting cavity;
an atomization core, wherein the atomization core is disposed within the mounting cavity, and a hollow atomization channel is formed in the atomization core; and
a sealing sleeve sleeved on an outer periphery of the porous support and enclosing the atomization core therein, wherein a side wall of the sealing sleeve is provided with a through liquid inlet hole.

2. The atomization core assembly according to claim 1, wherein an end of the porous support at which the sealing sleeve is mounted is further provided with a notch penetrating through the outer sidewall and communicating with the mounting cavity; and the atomization core comprises a liquid guide member and a heating member, and at least a portion of the liquid guide member extends into the notch.

3. The atomization core assembly according to claim 2, wherein the liquid guide member is configured as a sheet-shaped structure, and the sheet-shaped liquid guide member is enclosed to form the atomization channel, with at least one end located within the notch; an outer circumferential surface of the heating member is attached to an inner circumferential surface of the liquid guide member; and/or the porous support is a porous ceramic support.

4. The atomization core assembly according to claim 2, wherein the mounting cavity of the porous support is provided with an air inlet hole on a side facing away from the notch, the sealing sleeve is provided with an air outlet hole on a side proximate to the notch, and the air inlet hole communicates with the air outlet hole.

5. The atomization core assembly according to claim 2, wherein orthogonal projections of the liquid inlet hole and the notch onto the outer sidewall do not overlap.

6. The atomization core assembly according to claim 1, wherein the sealing sleeve comprises a cylindrical portion, a connecting portion, and a surrounding portion which are sequentially connected, the cylindrical portion and the surrounding portion are spaced apart and disposed on the same side of the connecting portion, the surrounding portion is disposed to surround a portion of an outer periphery of the cylindrical portion, the cylindrical portion is sleeved on the outer periphery of the porous support, and the cylindrical portion, the connecting portion, and the surrounding portion together enclose to form an insertion slot.

7. An assembly method for an atomization core assembly, wherein the assembly method comprises:
providing an atomization core and a rod body, and wrapping the atomization core around an outer periphery of the rod body;
providing a porous support having a mounting cavity, and inserting the rod body wrapped with the atomization core into the mounting cavity; and
providing a sealing sleeve, sleeving the sealing sleeve onto an outer periphery of the porous support, and pulling out the rod body.

8. The assembly method for an atomization core assembly according to claim 7, wherein the atomization core comprises a liquid guide member which is configured as a sheet-shaped structure, and the porous support is provided with a notch;
the wrapping the atomization core around an outer periphery of the rod body comprises: winding the sheet-shaped liquid guide member around the outer periphery of the rod body such that two ends of the sheet-shaped liquid guide member are attached to each other; and
the inserting the rod body wrapped with the atomization core into the mounting cavity comprises: aligning the two ends of the sheet-shaped liquid guide member with the notch and inserting the sheet-shaped liquid guide member into the mounting cavity; and cutting off a portion of an end portion of the sheet-shaped liquid guide member that extends out of the notch.

9. The assembly method for an atomization core assembly according to claim 7, wherein the porous support is configured as a porous ceramic support.

10. An atomizer, wherein the atomizer comprises the atomization core assembly according to any one of claims 1 to 6.

11. The atomizer according to claim 10, wherein the atomizer further comprises a base and an air guide tube, the air guide tube is sleeved on a part of the outer periphery of the sealing sleeve, the base is provided with a stepped recess, the sealing sleeve of the atomization core assembly is at least partially located within the stepped recess, and the sealing sleeve is configured to achieve a sealed connection between the base and the air guide tube.

12. The atomizer according to claim 11, wherein the atomizer further comprises a housing, the housing comprises a bottom housing, the housing is mounted around the outer periphery of the base and the air guide tube, the bottom housing is disposed on a side of the base and the air guide tube, the bottom housing is provided with an air vent hole, the base further comprises a blocking portion, the blocking portion is disposed on a surface of the stepped recess facing the atomization core assembly, and an orthogonal projection of the air vent hole onto the surface of the stepped recess facing the atomization core assembly falls within a range of the blocking portion, and the blocking portion is configured to prevent liquid from dripping into the air vent hole.

13. An atomization device, wherein the atomization device comprises the atomizer according to any one of claims 10 to 12.
